# EUROPÄISCHE PATENTSCHRIFT

(11) **EP 1 941 840 B1**
(45) Veröffentlichungstag und Bekanntmachung des Hinweises auf die Patenterteilung: **22.12.2010**
(21) Anmeldenummer: 08150024.1
(22) Anmeldetag: 03.01.2008
(51) Int. Cl.: A61B 17/28

(54) **Chirurgisches Instrument**
Surgical instrument
Instrument chirurgical

(30) Priorität: 08.01.2007 DE 102007001750
(43) Veröffentlichungstag der Anmeldung: 09.07.2008
(73) Patentinhaber: Aesculap AG, 78532 Tuttlingen (DE)
(72) Erfinder: Mayenberger, Rupert, 78239 Rielasingen (DE); Weissgraf, Jens Ole, 78532 Tuttlingen (DE)
(74) Vertreter: Hoeger, Stellrecht & Partner Patentanwälte

(56) Entgegenhaltungen:
- DE-U1- 29 701 170
- US-A- 4 712 545
- US-A- 5 603 723

## Beschreibung

Die vorliegende Erfindung betrifft ein chirurgisches Instrument mit einem proximalen und einem distalen Ende, mit einem am proximalen Ende angeordneten Handgriff, welcher mindestens ein erstes schwenkbar gelagertes Griffteil umfasst, mit mindestens einem am distalen Ende beweglich gelagerten Werkzeugelement, und mit einem beweglich am Instrument gelagerten Kraftübertragungsglied zum Übertragen einer Betätigungskraft vom mindestens einen ersten Griffteil auf das mindestens eine Werkzeugelement, wobei das Kraftübertragungsglied beweglich am mindestens einen ersten Griffteil gelagert ist.

Chirurgische Instrumente der eingangs beschriebenen Art werden beispielsweise in der Form von Rohrschaftinstrumenten bei minimalinvasiven chirurgischen Eingriffen verwendet. Ein Hauptproblem bei derartigen Instrumenten ist die Kraftübertragung vom mindestens einen ersten schwenkbar gelagerten Griffteil auf das Kraftübertragungsglied. In der Regel sind hierfür am beweglich gelagerten Griffteil zusätzliche Bauelemente erforderlich, um das Kraftübertragungsglied, beispielsweise eine Schub- und Zugstange, beweglich am mindestens einen ersten Griffteil zu lagern.

Aus der DE 297 01 170 U1 ist ein Instrumentengriff für ein chirurgisches Rohrschaftinstrument bekannt. Die US 5,603,723 offenbart ein chirurgisches Instrument, welches zu Reinigungszwecken zerlegbar ist. Und schließlich offenbart die US 4,712,545 weitere chirurgische Instrumente.

Es ist daher Aufgabe der vorliegenden Erfindung, ein chirurgisches Instrument der eingangs beschriebenen Art so zu verbessern, dass ein Aufbau des Instruments vereinfacht und eine optimale Krafteinleitung möglich wird.

Diese Aufgabe wird bei einem chirurgischen Instrument der eingangs beschriebenen Art erfindungsgemäß dadurch gelöst, dass bei Übertragung einer Zugkraft vom mindestens einen ersten Griffteil auf das Kraftübertragungsglied eine erste Zugkontaktfläche des Kraftübertragungsglieds an einer zweiten Zugkontaktfläche des ersten Griffteils flächig anliegt oder im Wesentlichen flächig anliegt und dass die erste und/oder die zweite Zugkontaktfläche gekrümmt sind und dass die erste und die zweite Zugkontaktfläche einen Ausschnitt einer inneren oder äußeren Kugeloberfläche bilden.

Die erfindungsgemäß vorgeschlagene Weiterbildung eines bekannten chirurgischen Instruments hat insbesondere den Vorteil, dass keine zusätzlichen Elemente erforderlich sind, um das Kraftübertragungsglied am mindestens einen ersten beweglich gelagerten Griffteil beweglich zu lagern. Das Kraftübertragungsglied und das mindestens eine erste Griffteil liegen bei Zugbeanspruchung des Kraftübertragungsglieds mit ihren ersten und zweiten Zugkontaktflächen direkt oder im Wesentlichen direkt aneinander an, vorzugsweise flächig, so dass maximal große Zugkräfte übertragen werden können. Dies ist insbesondere auch deshalb günstig, weil so auch Materialien zur Herstellung des ersten Griffteils und/oder des Kraftübertragungsglieds verwendet werden können, die eine geringere Eigenstabilität aufweisen als beispielsweise Instrumentenstahl. Denkbar sind hier insbesondere sterilisierbare Kunststoffe. Zudem ist bei einer gekrümmten Zugkontaktfläche auch eine optimale Verdrehung des Kraftübertragungsglieds um seine Längsachse relativ zum ersten Griffteil deutlich einfacher möglich.

Vorteilhaft ist es, dass die erste und/oder die zweite Zugkontaktfläche einen Ausschnitt einer inneren oder äußeren Kugeloberfläche bilden. Dies vereinfacht zum einen den Aufbau des Instruments und zum anderen ermöglicht es insbesondere auf einfache Weise nicht nur eine Schwenkbewegung des mindestens einen ersten Griffteils relativ zum Kraftübertragungsglied, sondern auch eine Verdrehbewegung des ersten Griffteils relativ zum Kraftübertragungsglied. Eine korrespondierende Ausbildung der ersten und/oder der zweiten Zugkontaktflächen, die vorzugsweise Kugeloberflächen definieren, ermöglichen insbesondere eine flächige Anlage der Zugkontaktflächen aneinander, wodurch eine optimale Krafteinleitung möglich ist, auch bei Materialien, die weniger stabil als Instrumentenstahl sind, beispielsweise Kunststoffen.

Vorteilhaft ist es, wenn das chirurgische Instrument einen langgestreckten rohrförmigen Schaft aufweist und wenn der Handgriff am proximalen Ende des Schafts angeordnet ist und wenn das mindestens eine Werkzeugelement am distalen Ende des Schafts angeordnet ist. Durch den langgestreckten rohrförmigen Schaft ist es möglich, das chirurgischen Instrument vorteilhaft bei minimalinvasiven chirurgischen Eingriffen einzusetzen, beispielsweise als endoskopisches Instrument. Der Schaft kann beispielsweise zur Aufnahme beweglicher Teile dienen und diese so schützen.

Günstig ist es, wenn das Kraftübertragungsglied beweglich im Schaft gelagert ist. Es wird so durch den Schaft geschützt. Ferner kann das Instrument mit dem Schaft in einen menschlichen oder tierischen Körper eingeführt werden, wobei bei eingeführtem Instrument keine Relativbewegung zwischen den Schaft umgebendem Gewebe und dem Schaft selbst erfolgt, eine Bewegung des ersten Griffteils führt lediglich zu einer Bewegung des mindestens einen ersten Werkzeugelements, welches am distalen Ende des Schafts angeordnet ist.

Besonders einfach wird der Aufbau des Instruments, wenn das Kraftübertragungsglied in Form einer Schub- und Zugstange ausgebildet ist.

Der Aufbau des Instruments vereinfacht sich weiter, wenn das mindestens eine Griffteil wellenfrei am Handgriff gelagert ist. Insbesondere kann so auf zusätzliche Bauteile verzichtet werden, die üblicherweise dazu dienen, das mindestens eine erste Griffteil am Handgriff zu lagern, beispielsweise ein eine Lagerwelle bildender zylindrischer Verbindungsstift.

Die Handhabung des Instruments wird deutlich verbessert, wenn der Handgriff ein unbeweglich am Handgriff angeordnetes zweites Griffteil umfasst. Eine Bedienperson kann sich so am zweiten Griffteil abstützen und das erste Griffteil relativ zum Handgriff bewegen.

Um den konstruktiven Aufwand des Instruments weiter zu verringern, ist es vorteilhaft, wenn der Handgriff und das zweite Griffteil einstückig ausgebildet sind. Es werden so keine Befestigungselemente benötigt, um das zweite Griffteil am Handgriff festzulegen.

Vorzugsweise ist das mindestens eine erste Griffteil einstückig ausgebildet. Beispielsweise könnte an diesem ein Teil einer Sperre angeordnet sein, die dann selbstverständlich auch vorzugsweise einstückig mit dem ersten Griffteil ausgebildet ist.

Besonders einfach wird die Konstruktion des chirurgischen Instruments, wenn der Handgriff insgesamt zweiteilig ausgebildet ist. Dies bedeutet, dass vorzugsweise ein feststehender Handgriffteil und ein beweglicher Handgriffteil vorgesehen sind, wobei zum Beispiel das zweite Griffteil am feststehenden Handgriffteil vorgesehen sein kann und der bewegliche zweite Handgriffteil das erste Griffteil umfasst.

Besonders leicht wird das Instrument und zudem kostengünstig herzustellen, wenn der Handgriff mindestens teilweise aus einem Kunststoff hergestellt ist.

Vorzugsweise ist er vollständig aus einem Kunststoff hergestellt. Dadurch eignet sich das Instrument auch hervorragend als Einweginstrument.

Grundsätzlich wäre es denkbar, das mindestens eine erste Griffteil am Handgriff verschiebbar anzuordnen. Vorteilhaft ist es jedoch, wenn das mindestens eine erste Griffteil um eine Schwenkachse verschwenkbar gelagert ist. Auf diese Weise lässt sich eine Handhabbarkeit des Instruments durch eine Bedienperson deutlich verbessern.

Günstigerweise verläuft die Schwenkachse durch einen nichtbeweglichen Teil des Handgriffs. Dies ermöglicht es insbesondere, das mindestens eine beweglich gelagerte erste Griffteil ohne eine Lagerwelle am Handgriff zu lagern.

Damit das chirurgische Instrument auch bei minimalinvasiven oder perkutanen Eingriffen eingesetzt werden kann, ist es vorteilhaft, wenn das chirurgische Instrument ein endoskopisches Instrument ist.

Um Handgriffe und Schäfte frei wählen zu können, ist es günstig, wenn der Handgriff und der Schaft miteinander lösbar verbindbar sind. Je nach chirurgischem Eingriff kann dann ein entsprechender Schaft mit einem dafür optimal geeigneten Handgriff verbunden werden. Zudem lässt sich das Instrument so deutlich leichter reinigen.

Gemäß einer bevorzugten Ausführungsform der Erfindung kann vorgesehen sein, dass der Handgriff und das mindestens eine erste Griffteil von einer Montagestellung, in welcher sie voneinander gelöst sind und/oder außer Eingriff stehen, in eine Arbeitsstellung, in welcher das mindestens eine erste Griffteil verschwenkbar gelagert ist, bringbar sind durch eine Relativbewegung des Handgriffs und des mindestens einen ersten Griffteils parallel zur Längsachse des Schafts. Durch diese Ausgestaltung wird eine Montage des Instruments deutlich vereinfacht.

Günstig ist es, wenn mindestens ein Sicherungsglied vorgesehen ist zum Sichern einer Lagerung des mindestens einen ersten Griffteils am Handgriff. Auf diese Weise kann verhindert werden, dass sich das mindestens eine erste Griffteil in unerwünschter Weise, beispielsweise wenn es sich in der Arbeitsstellung befindet, vom Handgriff löst.

Besonders einfach wird der Aufbau des Instruments, wenn das Kraftübertragungsglied das mindestens eine Sicherungsglied bildet oder umfasst. Es sind dann keine zusätzlichen Bauelemente erforderlich, um das mindestens eine erste beweglich gelagerte Griffteil, insbesondere in der Arbeitsstellung, am Handgriff sicher zu halten.

Vorteilhafterweise umfasst das mindestens eine erste Griffteil eine Sicherungsgliedaufnahme, in welcher das mindestens eine Sicherungsglied in der Arbeitsstellung mindestens teilweise gehalten ist. Zum Beispiel kann die Sicherungsgliedaufnahme einstückig mit dem Griffteil ausgebildet sein. So kann insbesondere auf zusätzliche Bauteile verzichtet werden. Zusätzliche Bauteile zu verwenden ist jedoch bekannt, erhöht den Montageaufwand des Instruments und zudem dessen Herstellungskosten. Ein solches zusätzliches Bauteil kann beispielsweise drehbar in einer Aufnahme gelagert sein oder verschiebbar. Bei bekannten Instrumenten wird hierfür ein verdicktes Ende des Kraftübertragungsglieds in das zusätzliche Bauteil, welches am mindestens einen ersten beweglich gelagerten Griffteil gehalten ist, eingehängt.

Günstig ist es, wenn das mindestens eine Sicherungsglied in der Sicherungsgliedaufnahme beweglich gelagert ist. Dadurch kann zum einen eine Relativbewegung des mindestens einen ersten Griffteils und des Handgriffs vereinfacht werden, ferner aber auch beispielsweise eine Verdrehbewegung des Handgriffs relativ zum Schaft und/oder zum Kraftübertragungsglied.

Besonders einfach wird der Aufbau des Instruments, wenn das Sicherungsglied ein verdicktes Ende aufweist oder ein verdicktes Ende des Kraftübertragungsglieds bildet und wenn das verdickte Ende in der Sicherungsgliedaufnahme gelagert ist. Insbesondere ist es so möglich, das Kraftübertragungsglied direkt mit dem mindestens einen ersten Griffteil zu verbinden.

Um eine Montage des Instruments zu vereinfachen, ist es vorteilhaft, wenn das Sicherungsglied in der Montagestellung mindestens teilweise in der Sicherungsgliedaufnahme einführbar ist und wenn das mit dem Sicherungsglied verbundene mindestens eine erste Griffteil und der Handgriff von der Montagestellung in die Bewegungsstellung überführbar sind. Bei der Montage wird also zunächst das Sicherungsglied in die Sicherungsgliedaufnahme ganz oder teilweise eingeführt und danach das mindestens eine erste Griffteil, welches bereits mit dem Sicherungsglied verbunden ist, mit dem Handgriff verbunden durch Überführen des Instruments von der Montagestellung in die Arbeitsstellung.

Gemäß einer bevorzugten Ausführungsform der Erfindung kann mindestens ein Anschlag vorgesehen sein zum Begrenzen einer Schwenkbewegung des mindestens einen ersten Griffteils am Handgriff. Dadurch kann insbesondere eine Beschädigung des am distalen Ende des Instruments gelagerten Werkzeugelements verhindert werden. Zudem lässt sich so auch eine Offen- und eine Schließstellung des mindestens einen Werkzeugelements definieren.

Vorzugsweise begrenzt der mindestens eine Anschlag eine Bewegung des mindestens einen ersten Griffteils und des mindestens einen zweiten Griffteils aufeinander zu und/oder voneinander weg. Insbesondere kann so eine Taktilität des Instruments verbessert werden, wenn beispielsweise extreme Relativstellungen des ersten und zweiten Griffteils mit extremen Stellungen des mindestens einen Werkzeugelements korrespondieren.

Besonders einfach wird der Aufbau des Instruments, wenn die Sicherungsgliedaufnahme den mindestens einen Anschlag umfasst und in einer Anschlagstellung mit dem mindestens einen Sicherungsglied in Kontakt steht. Auf diese Weise kann auf zusätzliche Anschläge verzichtet werden. Denkbar wäre es jedoch auch, weitere Anschläge vorzusehen, um eine Stabilität des Instruments zu erhöhen.

Vorzugsweise liegt ein Kugelmittelpunkt der Kugeloberfläche exzentrisch zur Schwenkachse. Auf diese Weise lässt sich eine Schwenkbewegung des mindestens einen ersten Griffteils einfach in eine translatorische Bewegung des Kraftübertragungsglieds übersetzen. Durch entsprechende Anordnung können so auch gewünschte Hebelverhältnisse eingestellt werden, um mit besonders geringem Kraftaufwand das mindestens eine erste Griffteil bewegen und die eingeleitete Kraft auf das mindestens eine Werkzeugelement zu übertragen.

Um stets eine optimale Krafteinleitung möglichst parallel zu einer Längsachse des Kraftübertragungsglieds auf dieses zu erreichen, ist es günstig, wenn der Kugelmittelpunkt infolge einer Schwenkbewegung des mindestens einen ersten Griffteils auf einer Kugelmittelpunktbahn relativ zur oder um die Schwenkachse wandert.

Vorteilhafterweise ist die Kugelmittelpunktbahn von der Schwenkachse weg weisend konvex gekrümmt. So lässt sich besonders einfach eine optimierte Krafteinleitung auf das Kraftübertragungsglied möglichst parallel zu dessen Längsachse sicherstellen.

Günstigerweise ist die Kugelmittelpunktbahn kreisförmig, wodurch eine Konstruktion des chirurgischen Instruments deutlich vereinfacht wird.

Gemäß einer bevorzugten Ausführungsform der Erfindung kann vorgesehen sein, dass die Krümmung der ersten und/oder zweiten Zugkontaktfläche derart gewählt ist, dass eine unter Zugbelastung auf die erste und/oder zweite Zugkontaktfläche wirkende Kraft parallel oder im Wesentlichen parallel zu einer Längsachse des Kraftübertragungsglieds in proximaler und/oder distaler Richtung gerichtet ist. Dadurch ist es möglich, im Prinzip die in das mindestens eine erste Griffteil eingeleitete Kraft ohne Verluste auf das Kraftübertragungsglied wirken zu lassen.

Die Krafteinleitung vom mindestens einen Griffteil auf das Kraftübertragungsglied wird weiter verbessert, wenn der Kugelmittelpunkt, unabhängig von einer Schwenkstellung des ersten Griffteils, auf der Längsachse des Kraftübertragungsglieds oder im Wesentlichen auf der Längsachse des Kraftübertragungsglieds liegt. Dies bedeutet mit anderen Worten, dass die Längsachse des Kraftübertragungsglieds senkrecht zur ersten und/oder zweiten Zugkontaktfläche orientiert ist, so dass die zu übertragende Zugkraft senkrecht in die Zugkontaktflächen eingeleitet wird.

Vorzugsweise sind die erste und die zweite Zugkontaktfläche korrespondierend zueinander ausgebildet. Dies bedeutet insbesondere, dass es so auf einfache Weise zu einer flächigen Anlage der ersten und der zweiten Zugkontaktfläche in der Arbeitsstellung aneinander kommen kann. So kann insbesondere auf zusätzliche Bauteile verzichtet werden, die bei herkömmlichen Instrumenten eine linienförmige Anlage von Teilen des Kraftübertragungsglieds an am mindestens einen ersten Griffteil beweglich gelagerten Bauelementen vermeiden, so dass also keine zusätzlichen Bauelemente erforderlich sind und das Kraftübertragungsglied in direktem Kontakt mit dem mindestens einen ersten beweglich gelagerten Griffteil stehen kann. Dies vereinfacht den Aufbau des Instruments, dessen Montage und somit auch dessen Herstellungskosten.

Der Aufbau des chirurgischen Instruments vereinfacht sich weiter, wenn das Sicherungsglied die erste Zugkontaktfläche umfasst und in distaler oder im Wesentlichen in distaler Richtung weist und wenn die Sicherungsgliedaufnahme die zweite Zugkontaktfläche umfasst und in proximaler oder im Wesentlichen in proximaler Richtung weist. Ein derart ausgebildetes Instrument benötigt keine zusätzlichen Bauelemente, um eine Kraft vom mindestens einen ersten Griffteil auf das Kraftübertragungsglied zu übertragen.

Besonders einfach wird der Aufbau des Instruments, wenn die Sicherungsgliedaufnahme in Form einer Nut ausgebildet ist. Diese lässt sich besonders einfach herstellen und mit einer Zugkontaktfläche ausstatten.

Um das Kraftübertragungsglied auf einfache Weise mit der Sicherungsgliedaufnahme in Eingriff bringen zu können, ist es günstig, wenn die Sicherungsgliedaufnahme einen in distaler Richtung weisenden Schlitz aufweist, welcher vom Kraftübertragungsglied durchsetzt ist. Beispielsweise kann ein verdicktes Ende des Kraftübertragungsglieds, welches ein Sicherungsglied bildet, so auf einfache Weise mit dem mindestens einen ersten Griffteil verbunden werden.

Eine optimale Beweglichkeit des mindestens einen ersten Griffteils relativ zum Handgriff wird dadurch sichergestellt, dass der Schlitz in einer Ebene senkrecht zur Schwenkachse verlaufend angeordnet ist.

Die Stabilität des Instruments wird insbesondere dadurch verbessert, dass die erste Zugkontaktfläche und die zweite Zugkontaktfläche jeweils zwei Zugkontaktflächenbereiche aufweisen, welche seitlich des Schlitzes und symmetrisch zu einer Symmetrieebene, welche senkrecht zur Schwenkachse verläuft, angeordnet sind. Dadurch kann insbesondere eine Krafteinleitung vom mindestens einen ersten Griffteil über die erste Zugkontaktfläche in flächiger Weise in die zweite Zugkontaktfläche des Kraftübertragungsglieds erfolgen. Durch die hinterschnittene Ausbildung der Sicherungsgliedaufnahme kann zudem eine seitliche Bewegung des Kraftübertragungsglieds und des mindestens einen ersten beweglich gelagerten Griffteils relativ zueinander parallel zur Schwenkachse verhindert werden.

Günstig ist es, wenn die Sicherungsgliedaufnahme seitlich hinterschnitten ist. So kann ein unbeabsichtigtes Lösen des Kraftübertragungsglieds und des mindestens einen ersten Griffteils voneinander verhindert werden.

Um zu verhindern, dass sich das in der Sicherungsgliedaufnahme gehaltene Sicherungsglied in der Arbeitsstellung von oder aus der Sicherungsgliedaufnahme lösen kann, ist es vorteilhaft, wenn die Sicherungsgliedaufnahme in der Arbeitsstellung durch mindestens eine innere Wandfläche des Eingriffs verschlossen ist. Es sind so insbesondere keine zusätzlichen Bauteile zum Verschließen der Sicherungsgliedaufnahme erforderlich. Vielmehr kann durch Überführen des Instruments von der Montagestellung in die Arbeitsstellung die Sicherungsgliedaufnahme automatisch durch die mindestens eine Wandfläche verschlossen werden.

Die nachfolgende Beschreibung einer bevorzugten Ausführungsform der Erfindung dient im Zusammenhang mit der Zeichnung der näheren Erläuterung. Es zeigen:
- Figur 1:: eine teilweise durchbrochene Seitenansicht eines chirurgischen Instruments;
- Figur 2:: eine vergrößerte, teilweise geschnittene Ansicht des Handgriffs des Instruments aus Figur 1;
- Figur 3:: eine vergrößerte Ansicht eines Kupplungsbereichs zwischen dem Handgriff und einem Kraftübertragungsglied; und
- Figur 4:: eine Schnittansicht längs Linie 4-4 in Figur 3.

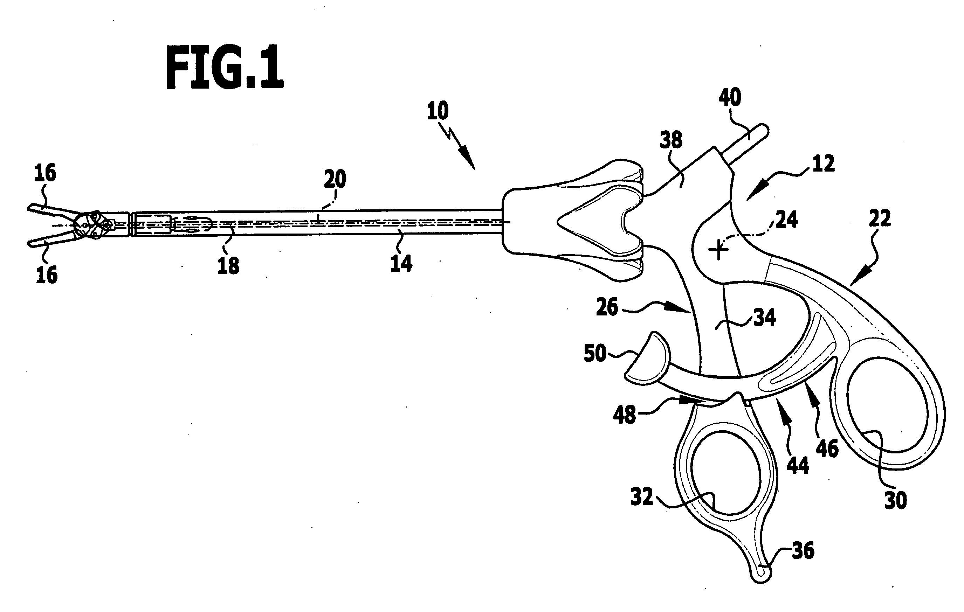

In Figur 1 ist ein insgesamt mit dem Bezugszeichen 10 versehenes chirurgisches Instrument in Form einer monopolaren endoskopischen Fasszange dargestellt. Es umfasst einen ein proximales Ende des Instruments 10 bildenden Handgriff 12 und einen verbindbar und relativ zu diesem verdrehbar angeordneten Schaft 14, an dessen distalem Ende zwei relativ zueinander bewegliche Maulteile 16 verschwenkbar gelagert sind.

Im Schaft 14 ist ein Kraftübertragungsglied 18 in Form einer Schub- und Zugstange parallel zu einer Längsachse 20 des Schafts 14 verschiebbar gelagert und distalseitig mit den Maulteilen 16 beweglich gekoppelt. Der Handgriff 12 umfasst ein erstes Griffteil 22, welches um eine quer zur Längsachse 20 verlaufende Drehachse 24 verschwenkbar gelagert ist, und ein zweites Griffteil 26, welches in etwa quer zur Längsachse 20 abstehend angeordnet ist. Ein proximales Ende 28 des Kraftübertragungsglieds 18 ist in einer nachfolgend im Detail näher beschriebenen Weise am ersten Griffteil 22 in der Nähe der Drehachse 24 beweglich gelagert, so dass infolge einer Verschwenkbewegung des ersten Griffteils 22 relativ zum zweiten Griffteil 26 das Kraftübertragungsglied 18 im Schaft 14 in distaler beziehungsweise proximaler Richtung bewegt werden kann, wodurch die Maulteile 16 aufeinander zu beziehungsweise voneinander weg bewegt werden.

Freie Enden der Griffteile 22 und 26 sind mit Ringen 30 und 32 zum Einführen von Fingern einer Bedienperson versehen. Vom Ring 32 des Griffteils 26 steht zudem, in etwa in Verlängerung eines ersten Griffabschnitts 34, ein Fingeranschlag 36 in Form eines länglichen Vorsprungs ab, der ein freies Ende des Griffteils 26 bildet. Der erste Griffabschnitt 34 erstreckt sich von einem Verbindungsabschnitt 38 des Handgriffs 12, der mit dem Schaft 14 verbindbar ist, im Wesentlichen quer zur Längsachse 20 vom Kraftübertragungsglied 18 weg. Des Weiteren steht vom Verbindungsabschnitt 38 in etwa unter einem Winkel von 45° in proximaler Richtung und von den Griffteilen 22 und 26 weg weisend ein Anschlusskontakt 40 ab, welcher mittels eines nicht dargestellten Anschlusskabels mit einer Stromquelle, beispielsweise einem Hochfrequenz-(HF)-Generator, verbunden werden kann. Der Anschlusskontakt 40 ist in nicht näher dargestellter Weise elektrisch leitend und mit dem Kraftübertragungsglied 18 verbunden, welches in elektrisch leitendem Kontakt mit mindestens einem der Maulteile 16 steht, so dass mit dem Instrument 10 beispielsweise Gewebe erfasst und gleichzeitig koaguliert werden kann.

Die Griffteile 22 und 26 und insbesondere die in diesen angeordneten Ringe 30 und 32 sind derart angeordnet, dass die Griffteile 22 und 26 im Wesentlichen eine die Längsachse 20 enthaltende, senkrecht zur Drehachse 24 verlaufende Bewegungsebene 42 definieren.

Eine Bewegung des ersten Griffteils 22 um die Drehachse 24 relativ zum zweiten Griffteil 26 ist nur eingeschränkt möglich. Grund hierfür ist eine eine Sperrvorrichtung bildende Sperre 44, welche ein erstes, am ersten Griffteil 22 angeordnetes Sperrglied 46 und eines zweites, am zweiten Griffteil 26 angeordnetes Sperrglied 48 umfasst. Der Handgriff 12, insbesondere die beiden Griffteile 22 und 26 sind vorzugsweise aus einem Kunststoff hergestellt. Die Sperrglieder 46 und 48 sind einstückig mit den Griffteilen 22 und 26 ausgebildet. Man kann auch sagen, die Sperrglieder 46 und 48 sind in das jeweilige Griffteil 22 beziehungsweise 26 integriert.

Die Sperre 44 verhindert in nicht näher dargestellter Weise eine Bewegung des ersten Griffteils 22 um die Drehachse 24 vom zweiten Griffteil 26 weg. Eine Bewegung des ersten Griffteils 22 auf das zweite Griffteil 26 zu ist jedoch möglich. Durch Betätigung eines ein freies Ende des ersten Sperrglieds 46 bildenden Betätigungsglieds 50 wird die Sperre 44 von ihrer Sperrstellung, die sie ohne Einwirkung äußerer Kräfte einnimmt, in eine Lösestellung überführt, in der das erste Griffteil 22 vom zweiten Griffteil 26 um die Drehachse 24 weg verschwenkt werden kann.

Zur beweglichen Lagerung des ersten Griffteils 22 am Verbindungsabschnitt 38 ist an letzterem eine Nut 52 vorgesehen, welche konzentrisch zur Drehachse 24 verlaufende Nutseitenflächen 54 und 56 aufweist. Die Nut 52 ist somit gekrümmt und endet an einem einen Anschlag bildenden Nutboden 58, der die Nut 52 in Richtung auf den Verbindungsabschnitt 38 hin begrenzt. Die Nut 52 ist in Richtung auf die Längsachse 20 hin geöffnet. An einem distalen Ende des ersten Griffteils 22 ist ein korrespondierend zur Nut 52 ausgebildeter Vorsprung 60 quer abstehend angeordnet, welcher in einer Arbeitsstellung, in der erste Griffteil 22 relativ zum zweiten Griffteil 26 um die Drehachse 24 verschwenkbar ist, ganz oder teilweise in die Nut 52 eingreift. Äußere Flächen des Vorsprungs 60 gleiten an den Nutseitenflächen 54 und 56 entlang, so dass eine Drehbewegung des ersten Griffteils 22 relativ zum zweiten Griffteil 26 um die Drehachse 24 durch den in die Nut 52 eingreifenden Vorsprung 60 definiert wird. Die Drehachse 24 verläuft quer zur Längsachse 20 durch einen feststehenden Teil des Handgriffs 12, nämlich einen Führungskörper 62, welcher ein proximales Ende des Handgriffs 12 bildet und die Nutseitenfläche 56 umfasst.

Das Kraftübertragungsglied 18 weist im Wesentlichen über seine gesamte Länge die Form eines zylindrischen Stabes auf. Im Bereich des Verbindungsabschnitts 38 ist das Kraftübertragungsglied 18 auf einem kurzen Abschnitt 64 im Außendurchmesser einstufig verringert. Dadurch wird ein am proximalen Ende 28 im Wesentlichen zylindrischer Körper ausgebildet, welcher ein Sicherungsglied 66 bildet. Eine den Abschnitt 64 umgebende Ringfläche des Sicherungsglieds 66, die in distaler Richtung weist, bildet eine erste Zugkontaktfläche 68. Die erste Zugkontaktfläche 68 bildet im Wesentlichen einen ringförmigen Ausschnitt einer Kugeloberfläche 70. Eine in proximaler Richtung weisende Endfläche 72 des Sicherungsglieds 66 ist von der ersten Zugkontaktfläche 68 weg weisend schwach konvex gekrümmt.

Am ersten Griffteil 22 ist an dessen distalem Ende benachbart des Vorsprungs 60 eine Nut 74 ausgebildet, die distalseitig parallel zur Bewegungsebene 42 mit einem Schlitz 76 versehen ist. Der Schlitz 76 ist schmaler als eine maximale Breite der Nut 74, so dass diese insgesamt hinterschnitten ausgebildet ist. Eine Breite des Schlitzes 76 entspricht in etwa einem Durchmesser des Abschnitts 64. Die Nut 74 ist in Richtung des Führungskörpers 62 geschlossen und weist einen Nutboden 78 auf, welcher eine Anschlagfläche definiert. Dem Nutboden 78 gegenüberliegend ist die Nut 52 geöffnet.

Koaxial zur Längsachse 20 ist der Verbindungsabschnitt 38 mit einer Bohrung 84 versehen, die sich proximalseitig zu einer Griffteilaufnahme 86 erweitert. Oberhalb der Längsachse 20 weist die Griffteilaufnahme 86 eine im Wesentlichen konzentrisch zur Drehachse 24 ausgebildete Innenfläche 88 auf, an welcher freie Enden von die Nut 74 definierenden Nutseitenwänden 90 und 92 bei einer Schwenkbewegung des ersten Griffteils 22 relativ zum zweiten Griffteil 26 entlang gleiten. Die Innenfläche 88 verschließt somit die Nut 74 in einer Arbeitsstellung.

Eine in proximale Richtung weisende Nutseitenfläche 80 der Nutseitenwand 90 bildet eine zweite Zugkontaktfläche, die ebenfalls im Wesentlichen einen Ausschnitt der Kugeloberfläche 70 definiert. Die der Nutseitenfläche 80 gegenüberliegende Nutseitenfläche 82 weist eine zur Krümmung der Endfläche 72 korrespondierende Krümmung auf. Die Nut 74 bildet eine Sicherungsgliedaufnahme, in welche das Sicherungsglied 66 des Kraftübertragungsglieds 18 einführbar ist, wenn das erste Griffteil 22 eine Montagestellung einnimmt, in welcher es vom Verbindungsabschnitt 38 gelöst ist. Der Abschnitt 64 durchsetzt nach dem Einführen des Sicherungsgliedes 66 in die Nut 74 den Schlitz 76. Um das Instrument 10 von der Montagestellung in die Arbeitsstellung zu überführen, wird das den in die Bohrung 84 eingeführten Schaft 14 durchsetzende und mit dem proximalen Ende des ersten Griffteils 22 verbundene Kraftübertragungsglied 18 in die Griffteilaufnahme 86 eingeschoben, bis die freien Enden der Nutseitenwände 90 und 92 an der Innenfläche 88 anliegen und der Vorsprung 60 in die Nut 52 um die Drehachse 24 hinein verschwenkt werden kann.

Ein Kugelmittelpunkt 94 der Kugeloberfläche 70 liegt oberhalb und proximalseitig der Drehachse 24 und außerhalb des Handgriffs 12. Infolge einer Schwenkbewegung des ersten Griffteils 22 relativ zum zweiten Griffteil 26 wandert der Kugelmittelpunkt 94 auf einer Kugelmittelpunktbahn 96, die von der Drehachse 26 weg weisend schwach konvex gekrümmt ist, das heißt ein minimaler Abstand der Kugelmittelpunktbahn 96 von der Drehachse 24 ist kleiner als ein minimaler Radius der Kugelmittelpunktbahn 96. Der Kugelmittelpunkt 94 wandert von einer proximalsten Stellung, in welcher er etwas unterhalb der Längsachse 20 liegt, in eine distalste Position, in der er etwas oberhalb der Längsachse 20 liegt, wenn das erste Griffteil 22 von einer maximal auf das zweite Griffteil 26 zu verschwenkten Stellung in eine vom zweiten Griffteil 26 maximal entfernte Stellung verschwenkt wird. In den Figuren ist die Relativstellung des ersten Griffteils 22 und des zweiten Griffteils 26 dargestellt, in welcher das erste Griffteil 22 maximal auf das zweite Griffteil 26 hin verschwenkt ist. Die erste Zugkontaktfläche 68 sowie die Nutseitenfläche 80 sind somit derart gekrümmt, dass eine unter Zugbelastung auf die erste und zweite Zugkontaktfläche wirkende Kraft parallel beziehungsweise im Wesentlichen parallel zur Längsachse 20 das Kraftübertragungsglied 18 in proximaler beziehungsweise distaler Richtung gerichtet ist. Dadurch kann eine in das erste Griffteil 22 eingeleitete Kraft praktisch vollständig auf das Kraftübertragungsglied 18 parallel zur Längsachse 20 übertragen werden. Folglich liegt auch der Kugelmittelpunkt 94, unabhängig von einer Schwenkstellung des ersten Griffteils 22, auf oder im Wesentlichen auf der Längsachse 20 des Kraftübertragungsglieds 18. Die Zugkontaktflächen liegen beim Instrument 10 flächig aneinander an, so dass kein zusätzliches Führungsglied, beispielsweise eine in eine entsprechende Nut eingesetzte Hülse zur Führung des Sicherungsglieds 66 am ersten Griffteil 22 erforderlich ist, wie dies bei aus dem Stand der Technik bekannten chirurgischen Instrumenten üblicherweise vorgesehen wird. Die Nutseitenfläche 82 ist ferner korrespondierend zur Endfläche 72 ausgebildet, so dass auch hier eine flächige Anlage in jeder Schwenkstellung gegeben ist.

Durch das Vorsehen des Schlitzes 76 ist die zweite Zugkontaktfläche, die durch die Nutseitenfläche 82 gebildet wird, in zwei Zugkontaktflächenbereiche aufgeteilt, die symmetrisch zur Bewegungsebene 42 angeordnet sind. Folglich liegt auch nicht die gesamte ringförmige erste Zugkontaktfläche 68 an der Nutzseitenfläche 80 an, sondern jeweils nur zwei symmetrisch zur Bewegungsebene 42 geformte Ringabschnitte der ersten Zugkontaktfläche 68.

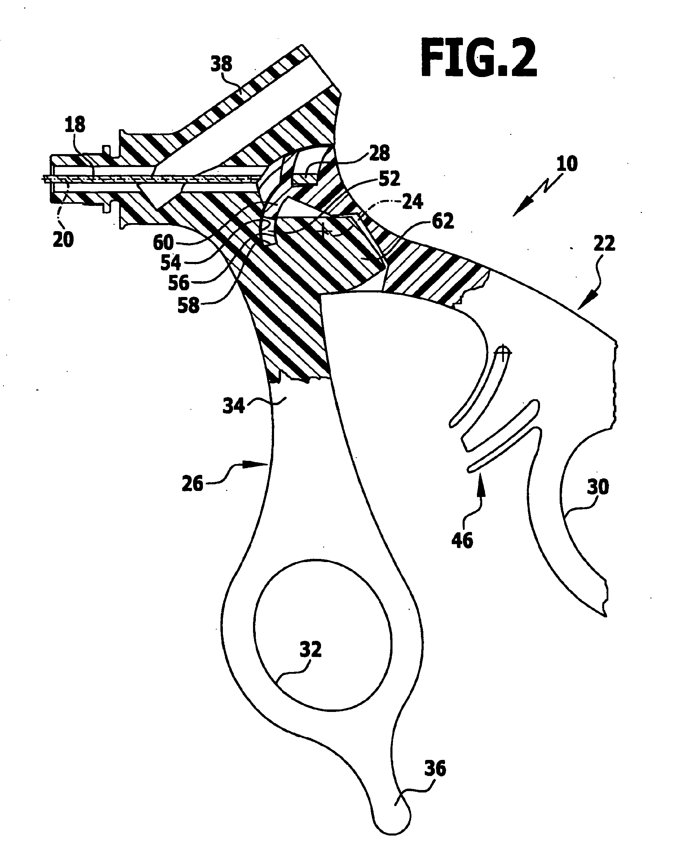

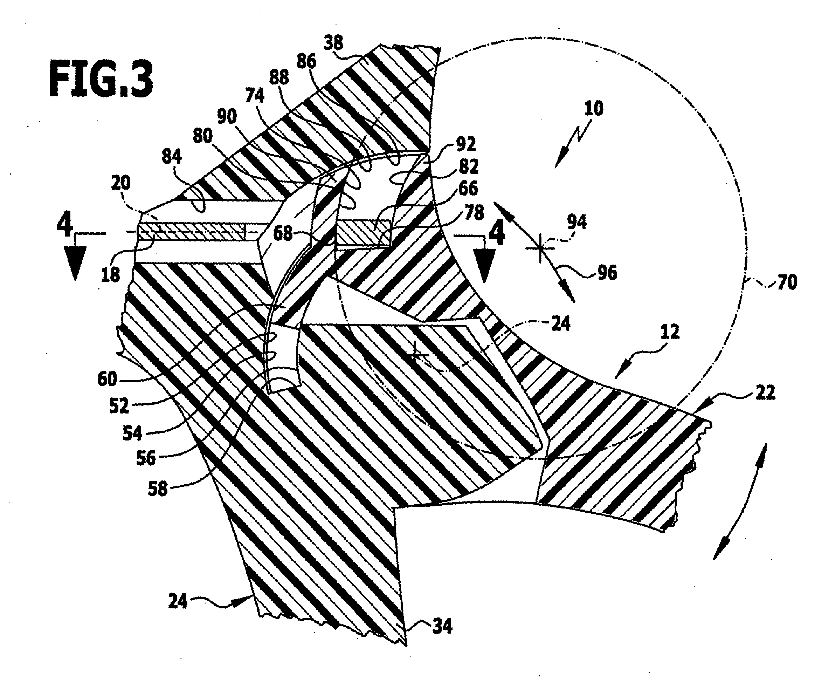

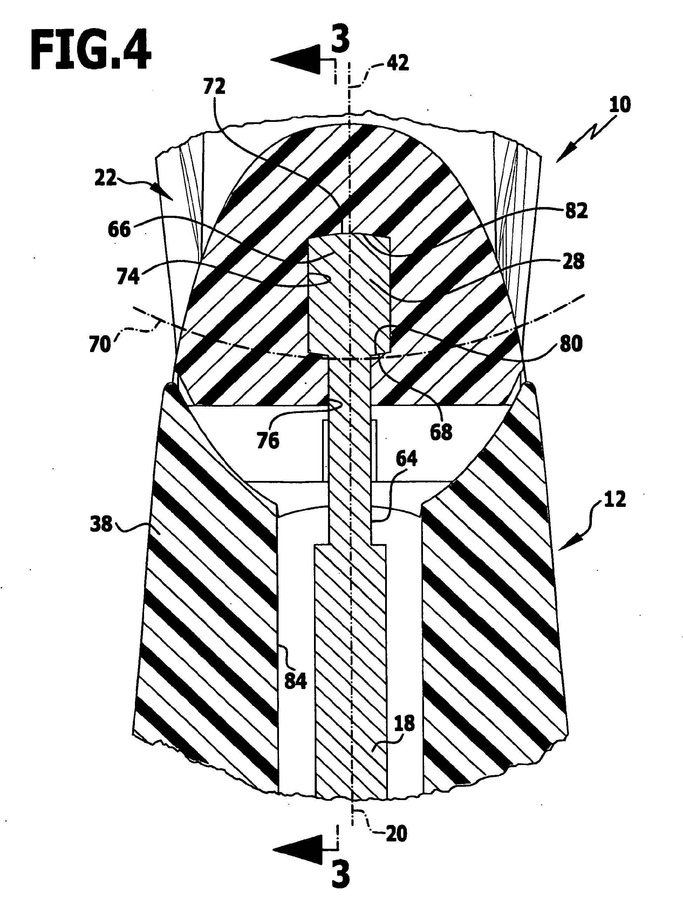

Der Handgriff 12 besteht somit im Wesentlichen aus zwei Teilen, nämlich dem ersten Griffteil 22 sowie dem einstückig mit dem zweiten Griffteil 26 ausgebildeten Verbindungsabschnitt 38. Eine Lagerung des ersten Griffteils 22 in der Arbeitsstellung, die in den Figuren 1 bis 4 dargestellt ist, wird allein durch das Zusammenwirken des Sicherungsglieds 66 mit der Nut 74 erreicht, somit sind keine zusätzlichen Bauelemente zur Lagerung des ersten Griffteils 22 am Handgriff 12 erforderlich.

Ferner können im Wesentlichen alle Teile des Instruments 10 aus einem Kunststoff hergestellt sein. Das Instrument 10 eignet sich somit hervorragend als Einwegprodukt.

Die Lage des Kugelmittelpunkts 94 wurde in dem in den Figuren dargestellten Ausführungsbeispiel so gewählt, dass er beim Auftreten der höchsten Kräfte direkt auf der Längsachse 20 liegt. Auf diese Weise wird auch die Biegebelastung der Zugstange minimiert.

Bei bevorzugten alternativen Ausführungsformen des Instruments 10 kann die Konstruktion des Handgriffs 12 auch dahingehend abgeändert werden, dass der Kugelmittelpunkt 94 in einer Mittelstellung des ersten Griffteils 22 zwischen einer maximal auf das zweite Griffteil 26 zu verschwenkten Stellung und einer maximal vom zweiten Griffteil 26 weg verschwenkten Stellung direkt oberhalb der Drehachse 24 auf der Längsachse 20 liegt. Der Kugelmittelpunkt 94 liegt dann in dieser Mittelstellung gleichzeitig auf einer Ebene senkrecht zur Längsachse 20, die die Drehachse 24 enthält. Die Kugelmittelpunktbahn 96 verläuft bei einer derartigen Ausgestaltung dann im Wesentlichen parallel zur Längsachse 20, so dass eine Einleitung von Schub- und Zugkräften vom ersten Griffteil 22 auf das Kraftübertragungsglied 18 praktisch in jeder Schwenkstellung des ersten Griffteils 22 relativ zum zweiten Griffteil 26 parallel zur Längsachse 20 erfolgt. Eine Ankopplung des Kraftübertragungsglieds 18 an das erste Griffteil 22 kann in diesem Fall dann noch weiter distalseitig der Drehachse 24 vorgesehen sein, als es dies bei dem in den Figuren 1 bis 4 dargestellten Instrument 10 der Fall ist.

## Patentansprüche

1. Chirurgisches Instrument (10) mit einem proximalen und einem distalen Ende, mit einem am proximalen Ende angeordneten Handgriff (12), welcher mindestens ein erstes schwenkbar gelagertes Griffteil (22) umfasst, mit mindestens einem am distalen Ende beweglich gelagerten Werkzeugelement (16), und mit einem beweglich am Instrument (10) gelagerten Kraftübertragungsglied (18) zum Übertragen einer Betätigungskraft vom mindestens einen ersten Griffteil (22) auf das mindestens eine Werkzeugelement (16), wobei das Kraftübertragungsglied (18) beweglich am mindestens einen ersten Griffteil (22) gelagert ist, **dadurch gekennzeichnet, dass** bei Übertragung einer Zugkraft vom mindestens einen ersten Griffteil (22) auf das Kraftübertragungsglied (18) eine erste Zugkontaktfläche (68) des Kraftübertragungsglieds (18) an einer zweiten Zugkontaktfläche (80) des ersten Griffteils (22) flächig anliegt oder im Wesentlichen flächig anliegt, dass die erste und/oder die zweite Zugkontaktfläche (68, 80) gekrümmt sind und dass die erste und die zweite Zugkontaktfläche (68, 80) einen Ausschnitt einer inneren oder äußeren Kugeloberfläche (70) bilden.

2. Chirurgisches Instrument nach Anspruch 1, **dadurch gekennzeichnet, dass** das chirurgische Instrument (10) einen langgestreckten rohrförmigen Schaft (14) aufweist und dass der Handgriff (12) am proximalen Ende des Schafts (14) angeordnet ist und dass das mindestens eine Werkzeugelement (16) am distalen Ende des Schafts (14) angeordnet ist.

3. Chirurgisches Instrument nach Anspruch 2, **dadurch gekennzeichnet, dass** das Kraftübertragungsglied (18) beweglich im Schaft (14) gelagert ist.

4. Chirurgisches Instrument nach einem der voranstehenden Ansprüche, **dadurch gekennzeichnet, dass** das Kraftübertragungsglied (18) in Form einer Schub- und Zugstange (18) ausgebildet ist.

5. Chirurgisches Instrument nach einem der voranstehenden Ansprüche, **dadurch gekennzeichnet, dass** das mindestens eine erste Griffteil (22) wellenfrei am Handgriff (12) gelagert ist.

6. Chirurgisches Instrument nach einem der voranstehenden Ansprüche, **dadurch gekennzeichnet, dass** der Handgriff (12) ein unbeweglich am Handgriff (12) angeordnetes zweites Griffteil (26) umfasst.

7. Chirurgisches Instrument nach Anspruch 6, **dadurch gekennzeichnet, dass** der Handgriff (12) und das zweite Griffteil (26) einstückig ausgebildet sind.

8. Chirurgisches Instrument nach einem der voranstehenden Ansprüche, **dadurch gekennzeichnet, dass** der mindestens eine erste Griffteil (22) einstückig ausgebildet ist.

9. Chirurgisches Instrument nach einem der voranstehenden Ansprüche, **dadurch gekennzeichnet, dass** der Handgriff (12) insgesamt zweiteilig ausgebildet ist.

10. Chirurgisches Instrument nach einem der voranstehenden Ansprüche, **dadurch gekennzeichnet, dass** der Handgriff (12) mindestens teilweise aus einem Kunststoff hergestellt ist.

11. Chirurgisches Instrument nach einem der voranstehenden Ansprüche, **dadurch gekennzeichnet, dass** das mindestens eine erste Griffteil (22) um eine Schwenkachse (24) verschwenkbar gelagert ist.

12. Chirurgisches Instrument nach Anspruch 11, **dadurch gekennzeichnet, dass** die Schwenkachse (24) durch einen nichtbeweglichen Teil des Handgriffs (12) verläuft.

13. Chirurgisches Instrument nach einem der voranstehenden Ansprüche, **dadurch gekennzeichnet, dass** das chirurgische Instrument (10) ein endoskopisches Instrument (10) ist.

14. Chirurgisches Instrument nach einem der voranstehenden Ansprüche, **dadurch gekennzeichnet, dass** der Handgriff (12) und der Schaft (14) miteinander lösbar verbindbar sind.

15. Chirurgisches Instrument nach Anspruch 14, **dadurch gekennzeichnet, dass** der Handgriff (12) und das mindestens eine erste Griffteil (22) von einer Montagestellung, in welcher sie voneinander gelöst sind und/oder außer Eingriff stehen, in eine Arbeitsstellung, in welcher das mindestens eine erste Griffteil (22) verschwenkbar gelagert ist, bringbar sind durch eine Relativbewegung des Handgriffs (12) und des mindestens einen ersten Griffteils (22) parallel zur Längsachse (20) des Schafts (14).

16. Chirurgisches Instrument nach einem der voranstehenden Ansprüche, **dadurch gekennzeichnet, dass** mindestens ein Sicherungsglied (66) vorgesehen ist zum Sichern einer Lagerung des mindestens einen ersten Griffteils (22) am Handgriff (12).

17. Chirurgisches Instrument nach Anspruch 16, **dadurch gekennzeichnet, dass** das Kraftübertragungsglied (18) das mindestens eine Sicherungsglied (66) bildet oder umfasst.

18. Chirurgisches Instrument nach einem der Ansprüche 16 oder 17, **dadurch gekennzeichnet, dass** das mindestens eine erste Griffteil (22) eine Sicherungsgliedaufnahme (74) umfasst, in welcher das mindestens eine Sicherungsglied (66) in der Arbeitsstellung mindestens teilweise gehalten ist.

19. Chirurgisches Instrument nach Anspruch 18, **dadurch gekennzeichnet, dass** das mindestens eine Sicherungsglied (66) in der Sicherungsgliedaufnahme (74) beweglich gelagert ist.

20. Chirurgisches Instrument nach einem der Ansprüche 16 bis 19, **dadurch gekennzeichnet, dass** das Sicherungsglied (18) ein verdicktes Ende (66) aufweist oder ein verdicktes Ende (66) des Kraftübertragungsglieds (18) bildet und dass das verdickte Ende (66) in der Sicherungsgliedaufnahme (74) gelagert ist.

21. Chirurgisches Instrument nach einem der Ansprüche 16 bis 20, **dadurch gekennzeichnet, dass** das Sicherungsglied (66) in einer Montagestellung, in welcher der Handgriff (12) und das mindestens eine erste Griffteil (22) voneinander gelöst sind und/oder außer Eingriff stehen, mindestens teilweise in die Sicherungsgliedaufnahme (74) einführbar ist und dass das mit dem Sicherungsglied (66) verbundene mindestens eine erste Griffteil (22) und der Handgriff (12) von der Montagestellung in die Arbeitsstellung überführbar sind.

22. Chirurgisches Instrument nach einem der voranstehenden Ansprüche, **dadurch gekennzeichnet, dass** mindestens ein Anschlag (58, 88) vorgesehen ist zum Begrenzen einer Schwenkbewegung des mindestens einen ersten Griffteils (22) am Handgriff (12).

23. Chirurgisches Instrument nach Anspruch 22, **dadurch gekennzeichnet, dass** der mindestens eine Anschlag (58, 88) eine Bewegung des mindestens einen ersten Griffteils (22) und des mindestens einen zweiten Griffteils (26) aufeinander zu und/oder voneinander weg begrenzt.

24. Chirurgisches Instrument nach einem der Ansprüche 22 oder 23, **dadurch gekennzeichnet, dass** das mindestens eine erste Griffteil (22) eine Sicherungsgliedaufnahme (74) umfasst, in welcher das mindestens eine Sicherungsglied (66) in der Arbeitsstellung mindestens teilweise gehalten ist und dass die Sicherungsgliedaufnahme (74) den mindestens einen Anschlag (58) umfasst und in einer Anschlagstellung mit dem mindestens einen Sicherungsglied (66) in Kontakt steht.

25. Chirurgisches Instrument nach einem der voranstehenden Ansprüche, **dadurch gekennzeichnet, dass** das mindestens eine erste Griffteil (22) um eine Schwenkachse (24) verschwenkbar gelagert ist und dass ein Kugelmittelpunkt (94) der Kugeloberfläche (70) exzentrisch zur Schwenkachse (24) liegt.

26. Chirurgisches Instrument nach Anspruch 25, **dadurch gekennzeichnet, dass** der Kugelmittelpunkt (94) infolge einer Schwenkbewegung des ersten Griffteils (22) auf einer Kugelmittelpunktbahn (96) relativ zur oder um die Schwenkachse (24) wandert.

27. Chirurgisches Instrument nach Anspruch 26, **dadurch gekennzeichnet, dass** die Kugelmittelpunktbahn (96) von der Schwenkachse (24) weg weisend konvex gekrümmt ist.

28. Chirurgisches Instrument nach einem der Ansprüche 26 oder 27, **dadurch gekennzeichnet, dass** die Kugelmittelpunktbahn (96) kreisförmig ist.

29. Chirurgisches Instrument nach einem der voranstehenden Ansprüche, **dadurch gekennzeichnet, dass** die Krümmung der ersten und/oder zweiten Zugkontaktfläche (68, 80) derart gewählt ist, dass eine unter Zugbelastung auf die erste und/oder zweite Zugkontaktfläche (68, 80) wirkende Kraft parallel oder im Wesentlichen parallel zu einer Längsachse (20) des Kraftübertragungsglieds (18) in proximaler und/oder distaler Richtung gerichtet ist.

30. Chirurgisches Instrument nach einem der Ansprüche 25 bis 29, **dadurch gekennzeichnet, dass** ein Kugelmittelpunkt (94) der Kugeloberfläche (70) exzentrisch zur Schwenkachse (24) liegt und dass der Kugelmittelpunkt (94), unabhängig von einer Schwenkstellung des ersten Griffteils (22), auf der Längsachse (20) des Kraftübertragungsglieds (18) oder im Wesentlichen auf der Längsachse (20) des Kraftübertragungsglieds (18) liegt.

31. Chirurgisches Instrument nach einem der voranstehenden Ansprüche, **dadurch gekennzeichnet, dass** die erste und die zweite Zugkontaktfläche (68, 80) korrespondierend zueinander ausgebildet sind.

32. Chirurgisches Instrument nach einem der Ansprüche 18 bis 31, **dadurch gekennzeichnet, dass** mindestens ein Sicherungsglied (66) vorgesehen ist zum Sichern einer Lagerung des mindestens einen ersten Griffteils (22) am Handgriff (12) und dass das Sicherungsglied (66) die erste Zugkontaktfläche (68) umfasst und in distaler oder im Wesentlichen in distaler Richtung weist und dass die Sicherungsgliedaufnahme (74) die zweite Zugkontaktfläche (80) umfasst und in proximaler oder im Wesentlichen in proximaler Richtung weist.

33. Chirurgisches Instrument nach einem der Ansprüche 18 bis 32, **dadurch gekennzeichnet, dass** das mindestens eine erste Griffteil (22) eine Sicherungsgliedaufnahme (74) umfasst, in welcher das mindestens eine Sicherungsglied (66) in der Arbeitsstellung mindestens teilweise gehalten ist und dass die Sicherungsgliedaufnahme (74) in Form einer Nut (74) ausgebildet ist.

34. Chirurgisches Instrument nach einem der Ansprüche 18 bis 33, **dadurch gekennzeichnet, dass** das mindestens eine erste Griffteil (22) eine Sicherungsglied aufnahme (74) umfasst, und dass die Sicherungsgliedaufnahme (74) einen in distaler Richtung weisenden Schlitz (76) aufweist, welcher vom Kraftübertragungsglied (18) durchsetzt ist.

35. Chirurgisches Instrument nach Anspruch 34, **dadurch gekennzeichnet, dass** das mindestens eine erste Griffteil (22) um eine Schwenkachse (24) verschwenkbar gelagert ist, und dass der Schlitz (76) in einer Ebene (42) senkrecht zur Schwenkachse (24) verlaufend angeordnet ist.

36. Chirurgisches Instrument nach einem der Ansprüche 34 oder 35, **dadurch gekennzeichnet, dass** das mindestens eine erste Griffteil (22) um eine Schwenkachse (24) verschwenkbar gelagert ist und dass die erste Zugkontaktfläche (68) und die zweite Zugkontaktfläche (80) jeweils zwei Zugkontaktflächenbereiche aufweisen, welche seitlich des Schlitzes (76) und symmetrisch zu einer Symmetrieebene (42), welche senkrecht zur Schwenkachse (24) verläuft, angeordnet sind.

37. Chirurgisches Instrument nach einem der Ansprüche 18 bis 36, **dadurch gekennzeichnet, dass** die Sicherungsgliedaufnahme (74) seitlich hinterschnitten ist.

38. Chirurgisches Instrument nach einem der Ansprüche 18 bis 37, **dadurch gekennzeichnet, dass** die Sicherungsgliedaufnahme (74) in der Arbeitsstellung durch mindestens eine innere Wandfläche (88) des Handgriffs verschlossen ist.

## Claims

1. Surgical instrument (10) with a proximal and a distal end, with a handle (12) which is arranged at the proximal end and comprises at least a first pivotably mounted handle part (22), with at least one tool element (16) moveably arranged at the distal end, and with a force transmission member (18) moveably mounted on the instrument (10) to transmit an actuating force from the at least one first handle part (22) to the at least one tool element (16), the force transmission member (18) being moveably mounted on the at least one handle part (22), **characterised in that** on transmission of a pulling force from at least a first handle part (22) to the force transmission member (18), a first pull contact face (68) of the force transmission member (18) abuts in planar, or substantially planar, manner against a second pull contact face (80) of the first handle part (22), **in that** the first and/or the second pull contact face (68, 80) are curved and **in that** the first and the second pull contact face (68, 80) form a section of an inner or outer spherical surface (70).

2. Surgical instrument according to claim 1, **characterised in that** the surgical instrument (10) has an elongate tubular shaft (14) and **in that** the handle (12) is arranged at the proximal end of the shaft (14) and **in that** the at least one tool element (16) is arranged at the distal end of the shaft (14).

3. Surgical instrument according to claim 2, **characterised in that** the force transmission member (18) is moveably mounted in the shaft (14).

4. Surgical instrument according to any one of the preceding claims, **characterised in that** the force transmission member (18) is configured in the form of a push and pull rod (18).

5. Surgical instrument according to any one of the preceding claims, **characterised in that** the at least one handle part (22) is mounted on the handle (12) without a spindle.

6. Surgical instrument according to any one of the preceding claims, **characterised in that** the handle (12) comprises a second handle part (26) immoveably arranged on the handle (12).

7. Surgical instrument according to claim 6, **characterised in that** the handle (12) and the second handle part (26) are configured in one piece.

8. Surgical instrument according to any one of the preceding claims, **characterised in that** the at least one first handle part (22) is configured in one piece.

9. Surgical instrument according to any one of the preceding claims, **characterised in that** the handle (12) is in total configured in two parts.

10. Surgical instrument according to any one of the preceding claims, **characterised in that** the handle (12) is produced at least partially from a plastics material.

11. Surgical instrument according to any one of the preceding claims, **characterised in that** the at least one first handle part (22) is pivotably mounted about a pivot axis (24).

12. Surgical instrument according to claim 11, **characterised in that** the pivot axis (24) passes through a non-moveable part of the handle (12).

13. Surgical instrument according to any one of the preceding claims, **characterised in that** the surgical instrument (10) is an endoscopic instrument (10).

14. Surgical instrument according to any one of the preceding claims, **characterised in that** the handle (12) and the shaft (14) are detachably connected to one another.

15. Surgical instrument according to claim 14, **characterised in that** the handle (12) and the at least one first handle part (22) can be brought from an assembly position, in which they are detached from one another and/or are disengaged, into a working position, in which the at least one first handle part (22) is pivotably mounted, by a relative movement of the handle (12) and the at least one first handle part (22) parallel to the longitudinal axis (20) of the shaft (14).

16. Surgical instrument according to any one of the preceding claims, **characterised in that** at least one securing member (66) is provided to secure a mounting of the at least one first handle part (22) on the handle (12).

17. Surgical instrument according to claim 16, **characterised in that** the force transmission member (18) forms or comprises the at least one securing member (66).

18. Surgical instrument according to either of claims 16 or 17, **characterised in that** the at least one first handle part (22) comprises a securing member receiver (74), in which the at least one securing member (66) is at least partially held in the working position.

19. Surgical instrument according to claim 18, **characterised in that** the at least one securing member (66) is moveably mounted in the securing member receiver (74).

20. Surgical instrument according to any one of claims 16 to 19, **characterised in that** the securing member (18) has a thickened end (66) or forms a thickened end (66) of the force transmission member (18) and **in that** the thickened end (66) is mounted in the securing member receiver (74).

21. Surgical instrument according to any one of claims 16 to 20, **characterised in that** the securing member (66), in an assembly position, in which the handle (12) and the at least one first handle part (22) are detached from one another and/or are disengaged, can be introduced at least partially into the securing member receiver (74), and **in that** the at least one first handle part (22) connected to the securing member (66) and the handle (12) can be transferred from the assembly position into the working position.

22. Surgical instrument according to any one of the preceding claims, **characterised in that** at least one stop (58, 88) is provided to limit a pivoting movement of the at least one first handle part (22) on the handle (12).

23. Surgical instrument according to claim 22, **characterised in that** the at least one stop (58, 88) limits a movement of the at least one first handle part (22) and the at least one second handle part (26) toward one another and/or away from one another.

24. Surgical instrument according to either of claims 22 or 23, **characterised in that** the at least one first handle part (22) comprises a securing member receiver (74), in which the at least one securing member (66) is at least partially held in the working position and **in that** the securing member receiver (74) comprises the at least one stop (58) and is in contact with the at least one securing member (66) in a stop position.

25. Surgical instrument according to any one of the preceding claims, **characterised in that** the at least one first handle part (22) is pivotably mounted about a pivot axis (24) and **in that** a sphere centre point (94) of the spherical surface (70) is eccentric with respect to the pivot axis (24).

26. Surgical instrument according to claim 25, **characterised in that** the sphere centre point (94) moves relative to or about the pivot axis (24) as a result of a pivoting movement of the first handle part (22) on a sphere centre point path (96).

27. Surgical instrument according to claim 26, **characterised in that** the sphere centre point path (96) is convexly curved directed away from the pivot axis (24).

28. Surgical instrument according to either of claims 26 or 27, **characterised in that** the sphere centre point path (96) is circular.

29. Surgical instrument according to any one of the preceding claims, **characterised in that** the curvature of the first and/or second pull contact face (68, 80) is selected in such a way that a force acting with pulling loading on the first and/or second pull contact face (68, 80) is directed parallel or substantially parallel to a longitudinal axis (20) of the force transmission member (18) in the proximal and/or distal direction.

30. Surgical instrument according to any one of claims 25 to 29, **characterised in that** a sphere centre point (94) of the spherical surface (70) is eccentric to the pivot axis (24) and **in that** the sphere centre point (94), independently of a pivoting position of the first handle part (22), is located on the longitudinal axis (20) of the force transmission member (18) or substantially on the longitudinal axis (20) of the force transmission member (18).

31. Surgical instrument according to any one of the preceding claims, **characterised in that** the first and the second pull contact face (68, 80) are configured corresponding with one another.

32. Surgical instrument according to any one of claims 18 to 31, **characterised in that** at least one securing member (66) is provided to secure a mounting of the at least one first handle part (22) on the handle (12) and **in that** the securing member (66) comprises the first pull contact face (68) and is oriented in the distal or substantially in the distal direction and **in that** the securing member receiver (74) comprises the second pull contact face (80) and is oriented in the proximal or substantially in the proximal direction.

33. Surgical instrument according to any one of claims 18 to 32, **characterised in that** the at least one first handle part (22) comprises a securing member receiver (74), in which the at least one securing member (66) is at least partially held in the working position and **in that** the securing member receiver (74) is configured in the form of a groove (74).

34. Surgical instrument according to any one of claims 18 to 33, **characterised in that** the at least one first handle part (22) comprises a securing member receiver (74), and **in that** the securing member receiver (74) has a slot (76) oriented in the distal direction, which has the force transmission member (18) passing through it.

35. Surgical instrument according to claim 34, **characterised in that** the at least one first handle part (22) is pivotably mounted about a pivot axis (24) and **in that** the slot (76) is arranged extending in a plane (42) perpendicular to the pivot axis (24).

36. Surgical instrument according to either of claims 34 or 35, **characterised in that** the at least one first handle part (22) is pivotably mounted about a pivot axis (24) and **in that** the first pull contact face (68) and the second pull contact face (80) each have two pull contact face regions, which are arranged to the side of the slot (76) and symmetrically with respect to a plane (42) of symmetry, which extends perpendicular to the pivot axis (24).

37. Surgical instrument according to any one of claims 18 to 36, **characterised in that** the securing member receiver (74) is laterally undercut.

38. Surgical instrument according to any one of claims 18 to 37, **characterised in that** the securing member receiver (74) is closed in the working position by at least one inner wall face (88) of the handle.

## Revendications

1. Instrument chirurgical (10) présentant une extrémité proximale et une extrémité distale, et comprenant une poignée de manoeuvre (12) qui est agencée à l'extrémité proximale et comporte au moins une première branche de préhension (22) montée pivotante, l'instrument comprenant également au moins un élément d'outil (16) monté mobile à l'extrémité distale, et un organe de transmission de force (18) monté de manière mobile sur l'instrument (10) et destiné à transmettre une force d'actionnement à partir de ladite au moins une première branche de préhension (22) au dit au moins un élément d'outil (16), l'organe de transmission de force (18) étant monté mobile sur ladite au moins une première branche de préhension (22)
**caractérisé en ce que** lors de la transmission d'une force de traction à partir de ladite au moins une première branche de préhension (22) à l'organe de transmission de force (18), une première surface de contact de traction (68) de l'organe de transmission de force (18) s'appuie selon une surface ou sensiblement selon une surface sur une deuxième surface de contact de traction (80) de la première branche de préhension (22), **en ce que** la première et/ou la deuxième surface de contact de traction (68, 80) sont courbées, et **en ce que** la première et la deuxième surfaces de contact de traction (68, 80) forment un secteur d'une surface sphérique intérieure ou extérieure (70).

2. Instrument chirurgical selon la revendication 1, **caractérisé en ce que** l'instrument chirurgical (10) présente un corps allongé (14) tubulaire de grande longueur, et **en ce que** la poignée de manoeuvre (12) est agencée à l'extrémité proximale du corps allongé (14), et **en ce que** ledit au moins un élément d'outil (16) est agencé à l'extrémité distale du corps allongé (14).

3. Instrument chirurgical selon la revendication 2, **caractérisé en ce que** l'organe de transmission de force (18) est monté mobile dans le corps allongé (14).

4. Instrument chirurgical selon l'une des revendications précédentes, **caractérisé en ce que** l'organe de transmission de force (18) est réalisé sous la forme d'une tige de poussée et de traction (18).

5. Instrument chirurgical selon l'une des revendications précédentes, **caractérisé en ce que** ladite au moins une première branche de préhension (22) est montée sans arbre sur la poignée de manoeuvre (12).

6. Instrument chirurgical selon l'une des revendications précédentes, **caractérisé en ce que** la poignée de manoeuvre (12) comprend une deuxième branche de préhension (26) agencée de manière non mobile sur la poignée de manoeuvre (12).

7. Instrument chirurgical selon la revendication 6, **caractérisé en ce que** la poignée de manoeuvre (12) et la deuxième branche de préhension (26) sont réalisées d'un seul tenant.

8. Instrument chirurgical selon l'une des revendications précédentes, **caractérisé en ce que** ladite au moins une première branche de préhension (22) est réalisée d'un seul tenant.

9. Instrument chirurgical selon l'une des revendications précédentes, **caractérisé en ce que** la poignée de manoeuvre (12) est réalisée en deux parties au total.

10. Instrument chirurgical selon l'une des revendications précédentes, **caractérisé en ce que** la poignée de manoeuvre (12) est fabriquée au moins en partie en une matière plastique.

11. Instrument chirurgical selon l'une des revendications précédentes, **caractérisé en ce que** ladite au moins une première branche de préhension (22) est montée de manière à pouvoir pivoter autour d'un axe de pivotement (24).

12. Instrument chirurgical selon la revendication 11, **caractérisé en ce que** l'axe de pivotement (24) s'étend à travers une partie non mobile de la poignée de manoeuvre (12).

13. Instrument chirurgical selon l'une des revendications précédentes, **caractérisé en ce que** l'instrument chirurgical (10) est un instrument endoscopique (10).

14. Instrument chirurgical selon l'une des revendications précédentes, **caractérisé en ce que** la poignée de manoeuvre (12) et le corps allongé (14) peuvent être reliés mutuellement de manière amovible.

15. Instrument chirurgical selon la revendication 14, **caractérisé en ce que** la poignée de manoeuvre (12) et ladite au moins une première branche de préhension (22) peuvent être amenées d'une position de montage, pour laquelle elles sont désolidarisées l'une de l'autre et/ou sont hors de prise réciproque, dans une position de travail dans laquelle ladite au moins une première branche de préhension (22) est montée pivotante, grâce à un mouvement relatif de la poignée de manoeuvre (12) et de ladite au moins une première branche de préhension (22) parallèlement à l'axe longitudinal (20) du corps allongé (14).

16. Instrument chirurgical selon l'une des revendications précédentes, **caractérisé en ce qu'**il est prévu au moins un organe de sécurité (66) pour sécuriser un montage de ladite au moins une première branche de préhension (22) sur la poignée de manoeuvre (12).

17. Instrument chirurgical selon la revendication 16, **caractérisé en ce que** l'organe de transmission de force (18) forme ou comprend ledit au moins un organe de sécurité (66).

18. Instrument chirurgical selon l'une des revendications 16 ou 17, **caractérisé en ce que** ladite au moins une première branche de préhension (22) présente un logement de réception d'organe de sécurité (74), dans lequel ledit au moins un organe de sécurité (66) est retenu au moins partiellement, dans la position de travail.

19. Instrument chirurgical selon la revendication 18, **caractérisé en ce que** ledit au moins un organe de sécurité (66) est monté mobile dans le logement de réception d'organe de sécurité (74).

20. Instrument chirurgical selon l'une des revendications 16 à 19, **caractérisé en ce que** l'organe de sécurité (18) présente une extrémité évasée (66) ou forme une extrémité évasée (66) de l'organe de transmission de force (18), et **en ce que** l'extrémité évasée (66) est montée ou logée dans le logement de réception d'organe de sécurité (74).

21. Instrument chirurgical selon l'une des revendications 16 à 20, **caractérisé en ce que** l'organe de sécurité (66), dans une position de montage pour laquelle la poignée de manoeuvre (12) et ladite au moins une première branche de préhension (22) sont désolidarisées l'une de l'autre et/ou sont hors de prise réciproque, peut être introduit au moins partiellement dans le logement de réception d'organe de sécurité (74), et **en ce que** ladite au moins une première branche de préhension (22) reliée à l'organe de sécurité (66) et la poignée de manoeuvre (12) peuvent être transférées de la position de montage dans la position de travail.

22. Instrument chirurgical selon l'une des revendications précédentes, **caractérisé en ce qu'**il est prévu au moins une butée (58, 88) pour limiter un mouvement de pivotement de ladite au moins une première branche de préhension (22) sur la poignée de manoeuvre (12).

23. Instrument chirurgical selon la revendication 22, **caractérisé en ce que** ladite au moins une butée (58, 88) limite un mouvement de ladite au moins une première branche de préhension (22) et de ladite au moins une deuxième branche de préhension (26) lorsque celles-ci se déplacent l'une vers l'autre et/ou s'écartent l'une de l'autre.

24. Instrument chirurgical selon l'une des revendications 22 ou 23, **caractérisé en ce que** ladite au moins une première branche de préhension (22) présente un logement de réception d'organe de sécurité (74) dans lequel ledit au moins un organe de sécurité (66) est, dans la position de travail, retenu au moins partiellement, et **en ce que** le logement de réception d'organe de sécurité (74) englobe ladite au moins une butée (58) et, dans une position de butée, est en contact avec ledit au moins un organe de sécurité (66).

25. Instrument chirurgical selon l'une des revendications précédentes, **caractérisé en ce que** ladite au moins une première branche de préhension (22) est montée de manière à pouvoir pivoter autour d'un axe de pivotement (24), et **en ce qu'**un centre de sphère (94) de la surface sphérique (70) se situe dans une position excentrée par rapport à l'axe de pivotement (24).

26. Instrument chirurgical selon la revendication 25, **caractérisé en ce que** suite à un mouvement de pivotement de la première branche de préhension (22), le centre de sphère (94) se déplace sur une trajectoire de centre de sphère (96) par rapport à de l'axe de pivotement (24) ou autour de celui-ci.

27. Instrument chirurgical selon la revendication 26, **caractérisé en ce que** la trajectoire de centre de sphère (96) est d'une courbure convexe en s'éloignant de l'axe de pivotement (24).

28. Instrument chirurgical selon l'une des revendications 26 ou 27, **caractérisé en ce que** la trajectoire de centre de sphère (96) est de forme circulaire.

29. Instrument chirurgical selon l'une des revendications précédentes, **caractérisé en ce que** la courbure de la première et/ou de la deuxième surface de contact de traction (68, 80) est choisie de façon à ce qu'une force agissant sur la première et/ou la deuxième surface de contact de traction (68, 80) sous sollicitation de traction, soit orientée parallèlement ou sensiblement de manière parallèle à un axe longitudinal (20) de l'organe de transmission de force (18), dans la direction proximale et/ou distale.

30. Instrument chirurgical selon l'une des revendications 25 à 29, **caractérisé en ce qu'**un centre de sphère (94) de la surface sphérique (70) est dans une position excentrée par rapport à l'axe de pivotement (24), et **en ce que** le centre de sphère (94) se situe, indépendamment d'une position de pivotement de la première branche de préhension (22), sur l'axe longitudinal (20) de l'organe de transmission de force (18) ou sensiblement sur l'axe longitudinal (20) de l'organe de transmission de force (18).

31. Instrument chirurgical selon l'une des revendications précédentes, **caractérisé en ce que** la première et la deuxième surface de contact de traction (68, 80) sont de configuration correspondante ou complémentaire.

32. Instrument chirurgical selon l'une des revendications 18 à 31, **caractérisé en ce qu'**il est prévu au moins un organe de sécurité (66) pour sécuriser un montage de ladite au moins une première branche de préhension (22) sur la poignée de manoeuvre (12), et **en ce que** l'organe de sécurité (66) comprend la première surface de contact de traction (68) et est orienté vers la direction distale ou sensiblement distale, et **en ce que** le logement de réception d'organe de sécurité (74) comprend la deuxième surface de contact de traction (80) et est orienté vers la direction proximale ou sensiblement proximale.

33. Instrument chirurgical selon l'une des revendications 18 à 32, **caractérisé en ce que** ladite au moins une première branche de préhension (22) comporte un logement de réception d'organe de sécurité (74) dans lequel ledit au moins un organe de sécurité (66) est retenu au moins partiellement, dans la position de travail, et **en ce que** le logement de réception d'organe de sécurité (74) est réalisé sous la forme d'une rainure (74).

34. Instrument chirurgical selon l'une des revendications 18 à 33, **caractérisé en ce que** ladite au moins une première branche de préhension (22) comporte un logement de réception d'organe de sécurité (74), et **en ce que** le logement de réception d'organe de sécurité (74) présente une fente (76) orientée dans la direction distale et traversée par l'organe de transmission de force (18).

35. Instrument chirurgical selon la revendication 34, **caractérisé en ce que** ladite au moins une première branche de préhension (22) est montée de manière à pouvoir pivoter autour d'un axe de pivotement (24), et **en ce que** la fente (76) est agencée dans un plan (42) s'étendant perpendiculairement à l'axe de pivotement (24).

36. Instrument chirurgical selon l'une des revendications 34 ou 35, **caractérisé en ce que** ladite au moins une première branche de préhension (22) est montée de manière à pouvoir pivoter autour d'un axe de pivotement (24), et **en ce que** la première surface de contact de traction (68) et la deuxième surface de contact de traction (80) présentent chacune deux zones de surface de contact de traction, qui sont agencées latéralement à la fente (76) et symétriquement par rapport à un plan de symétrie (42) s'étendant perpendiculairement à l'axe de pivotement (24).

37. Instrument chirurgical selon l'une des revendications 18 à 36, **caractérisé en ce que** le logement de réception d'organe de sécurité (74) est d'une configuration à contre dépouille latérale.

38. Instrument chirurgical selon l'une des revendications 18 à 37, **caractérisé en ce que** le logement de réception d'organe de sécurité (74) est fermé, dans la position de travail, par au moins une surface de paroi intérieure (88) de la poignée de manoeuvre.
